# EUROPEAN PATENT SPECIFICATION

(11) **EP 2 900 826 B1**
(45) Date of publication and mention of the grant of the patent: **13.07.2016**
(21) Application number: 13785666.2
(22) Date of filing: 27.09.2013
(51) Int. Cl.: C12P 7/18, B01D 11/04, C07C 29/76, C07C 29/80

(54) **A METHOD FOR ISOLATION OF PROPANE-1,3-DIOL FROM POST-FERMENTATION BROTH OBTAINED BY BIOCONVERSION**
VERFAHREN ZUR ISOLIERUNG VON PROPAN-1,3-DIOL AUS EINER BRÜHE NACH FERMENTATION HERGESTELLT DURCH BIOCONVERSION
UNE MÉTHODE D'ISOLEMENT DE PROPANE-1 ,3-DIOL OBTENU PAR BIOCONVERSION D'UN BOUILLON APRÈS FERMENTATION

(30) Priority: 28.09.2012 PL 40097912
(43) Date of publication of application: 05.08.2015
(73) Proprietor: Prochimia Surfaces Sp. Z O.O., 81-823 Sopot (PL)
(72) Inventor: LINDSTAEDT, Agnieszka, 84-217 Jelenska Huta (PL); WITT, Dariusz, PL-80-299 Gdansk (PL); PUZEWICZ-BARSKA, Joanna, PL-81-578 Gdynia (PL); BARSKI, Piotr, PL-81-823 Sopot (PL)
(74) Representative: Wazynska, Miroslawa
(86) International application number: PCT/PL2013/000124
(87) International publication number: WO 2014/051448

(56) References cited:
- US-A1- 2012 184 783
- US-B2- 7 056 439
- DATABASE WPI Week 200420 Thomson Scientific, London, GB; AN 2004-204156 XP002719064, & CN 1 460 671 A (UNIV DALIAN POLYTECHNIC) 10 December 2003 (2003-12-10) cited in the application
- GUNEET KAUR ET AL: "Advances in biotechnological production of 1,3-propanediol", BIOCHEMICAL ENGINEERING JOURNAL, ELSEVIER, AMSTERDAM, NL, vol. 64, 7 March 2012 (2012-03-07), pages 106-118, XP028411971, ISSN: 1369-703X, DOI: 10.1016/J.BEJ.2012.03.002 [retrieved on 2012-03-16]
- ZHI-LONG XIU ET AL: "Present state and perspective of downstream processing of biologically produced 1,3-propanediol and 2,3-butanediol", APPLIED MICROBIOLOGY AND BIOTECHNOLOGY, SPRINGER, BERLIN, DE, vol. 78, no. 6, 5 March 2008 (2008-03-05), pages 917-926, XP019586363, ISSN: 1432-0614

## Description

The present invention relates to a method for isolation of propane-1,3-diol from post-fermentation broth obtained by bioconversion.
Propane-1,3-diol is mainly used, in particular as monomer, to build polymer PTT (polytrimethylene terephthalate) used in production of fibers, carpets, textiles, for sanitary and medical applications, and as a packaging and/or structural material.
As used herein "bioconversion" is a synonym of any fermentation process leading to enzymatic/biochemical way of production of propane-1,3-diol.

US 7 056 439 B2 discloses a method for extraction of propane-1,3-diol from fermentation broth with alcohols (pentanol, propanol, hexanol, oleyl alcohol), ketones (4-methyl-2-pentanone), esters (isopropyl acetate, tributyl phosphate), oleic acid, soybean oil, alkanes (n-hexane) and mixtures thereof. This method requires multiple extraction with different solvents and reverse extraction to aqueous phase. Maximum purity of isolated propane-1,3-diol is 95-98.5%.
US 5 008 473 describes a method for isolation of propane-1,3-diol with cyclohexane. This method is not suitable for mixtures obtained in biotechnological processes and containing glycerol.
From EP 1 720 814 B1 a method is known for isolation of hydrophilic organic compounds, including propane-1.3-diol, with glycol ether at a temperature and then as a result of heating to a higher temperature to obtain an aqueous extract with hydrophilic compound and ether rafinate. The method is based on extraction with ether glycols and a phenomenon specific for them of decreasing the ether solubility in aqueous phase with the increase of temperature. The method discloses also addition of organic solvents in order to further decrease the glycol ethers solubility in aqueous phase during reverse extraction step to aqueous phase. Among additives some ketones are here mentioned In this method organic solvent was not used alone for extraction per se. This function was fulfilled by glycol ether, that by nature, forces the temperature increase and reverse extraction to aqueous phase. The whole solution thus constitutes the concentration of propane-1,3-diol in aqueous phase, as in US 7 056 439 B2.

US 20120184783 discloses a process for purification of particular diols, including propane-1,3-diol, from diols mixture by extraction with an organic solvent or a mixture of organic solvents. A proper selection of solvent mixture allows to recover a selected diol group (comprising three, four and five carbon atoms), from the starting mixture, where content of water is max. 50 wt.%. Presented method is not effective however to separate diols from post-fermentation broth with glycerol content of less than 5 wt.% nor selective isolation of propane-1,3-diol. The method fails to complete the use of four-carbon ketons C4 (2-butanone).
CN101012151A describes a method of propane-1,3-diol isolation from post-fermentation broth containing trimethylene glycol in the range of (30-700g/L) consisting in an initial saturation with inorganic salts (10-100%) and organic solvent addition for extraction. The method concerns specific fermentation broth with trimetylene glycol and comprises a technically inconvenient and expensive step of inorganic salt addition. Application of more than three- carbon ketons (C3) is omitted. CN 1907929 discloses a method of isolation of propane-1,3-diol from fermentation broth containing trimethylene glycol by extraction combined with chemical reaction. The recovery of propane-1,3-diol is made after hydrolysis and distillation. This is a costly and multistage process which reduces efficiency thereof and has negative impact on its profitability.
Similar recovery technique is presented by Malinowski et al. (Biotechnol. Prog., 16: 76-79. 2000). This extraction is based on isolation of propane-1,3-diol by extraction with aldehydes, as reactants, converting propane-1,3-diol into alkyl dioxane and the product is next extracted with organic solvents (toluene, o-xylene, ethylbenzene).

Process of propane-1,3-diol extraction from post-fermentation broth with mixture of ethyl acetate and ethanol is known in the art from Separation Science and Technology, 45: 541-547, 2010.

Propane-1,3-diol can also be recovered by continuous liquid-liquid extraction (Biotechnol. Tech., 13: 127-130, 1999) however, this process is reported to be of limited efficiency due to a low distribution ratio of propane-1,3-diol in organic solvents.

CN1460671A discloses a step of thermal concentration for reduction of volume of the post-fermentation broth (containing the product) and biomass removal step by precipitation with organic solvent. Liquid-liquid extraction and aqueous/organic phase mixing are not mentioned there. Mixing is performed only during biomass washing after precipitation. Methyl-ethyl ketone is added, as a precipitating agent, only to remove the biomass, but not as the extractant. This is evidenced by absence of phase distribution (aqueous/organic) and extraction of the supernatant was not performed.

The solution of CN1460671A describes a process for distillation of mixture of water (from post-fermentation broth) and an organic solvent (used for precipitation of biomass). The solution described lacks in the critical liquid-liquid extraction process.

In the solution of the invention the method has no concentration or precipitation steps and organic solvent is used for liquid-liquid extraction and not for biomass precipitation.

In the present invention, contrary to the solution of CN1460671A, the extracted substance (propane-1,3-diol) in the liquid-liquid extraction becomes distributed between a primary solution (post-fermentation broth) and a secondary solution (organic solvent - methyl-ethyl ketone, with which the key compound is extracted). As a result, a rafinate is obtained (a solution basically without propane-1,3-diol) and an extract, containing propane-1,3-diol. Solely the extract (not all the aqueous phase - supernatant from steps 2 and 3 as in CN1460671A) is further purified (eg. subjected to distillation) to acquire pure final product and solvent (extractant).

According to the invention, at the step of solvent recovery and final product purification, due to efficient counter-current extraction the water content is minimized to 12% of water solubilized in methyl-ethyl ketone because of mixing during liquid-liquid extraction process.

EP 0 261 554 A discloses extraction of propane-1,3-diol with cyclohexane while the present invention concerns propane-1,3-diol isolation in continuous liquid-liquid extraction from post-fermentation broth (after biomass removal) by means of 2-butanone and final product distillation.

EP1103618B1 is based on a known method of ion exchange chromatography, where propane-1,3-diol is isolated using a cationic resin and in the next step a column of activated carbon and a weak basic anionic resin or cationic resin and strong basic anionic resin. Thus it differs from the invention in which propane-1,3-diol is isolated in the continuous liquid-liquid extraction from post-fermentation broth (after biomass removal) by means of 2-butanone and final product distillation.

US6603048B1 discloses a method of isolation of propane-1,3-diol from post-fermentation broth, which comprises propane-1,3-diol and other fermentation co-using zeolites as molecular sieves: products, (inter alia, glycerol), using molecular sieves, which is zeolites. The process is an example of industrial use of propane-1,3-diol recovery method from the post-fermentation broth. Despite it proposed industrial use, the method uses an expensive separation medium and separation of products on a semi-continuous basis that generates additional capital and production expenses.

CN101497556A discloses a solid super acidic catalyst method as a method for Isolation of propane-1,3-diol followed by aldol condensation reaction, and rectification.

The present invention relates to the method of continuous liquid-liquid extraction from post-fermentation broth (after biomass removal) with 2-butanone, with no use of catalyst reaction and aldol reaction.

Up to now there is no continuous method known for extraction of propane-1,3-diol directly from post-fermentation broth, obtained from almost any fermentation process to obtain propane-1,3-diol, by means of 2-butanone.

The inventors faced to a need for obtaining pure propane-1,3-diol have developed a simple and inexpensive method for isolation of propane-1,3-diol from post-fermentation broth.

The object of the present invention is a method for isolation of propane-1,3-diol from the post-fermentation broth consisting in propane-1,3-diol extraction by means of a mixture of organic solvents, characterized in that propane-1,3-diol is isolated, after previous biomass removal from post-fermentation broth, with 2-butanone that is present in the organic solvent mixture. The method is carried out preferably continuously or stepwise and the organic solvent used in the extraction method is used repeatedly. The content of 2-butanone in organic solvents mixture is minimum 10%, and the volume ratio of post-fermentation broth, containing propane-1,3-diol, to the mixture of organic solvents is minimum 1:3. After previous biomass removal the isolation of propane-1,3-diol is conducted without salting out or concentration of post-fermentation broth. The method of the invention is also characterized in that isolation of propane-1,3-diol by means of organic solvent mixture, is followed by distillation under vacuum to obtain highly pure propane-1,3-diol. Preferably first biomass is removed from the post-fermentation broth comprising propane-1,3-diol by inter alia: ultrafiltration, filtration, centrifugation or sedimentation, and then the extraction of propane-1,3-diol with organic solvent mixture is carried out.

The method of the invention of propane-1,3-diol recovery from post-fermentation broth is based on continuous or stepwise propane-1,3-diol extraction with an organic solvent, as 2-butanone.

An advantage of the method of the invention is the increased ratio of recovery of propane-1,3-diol from post-fermentation broth obtained from any fermentation process leading to production of propane-1,3-diol, as a main product. As the source of carbon, there can be employed glycerol or carbohydrates.

The post-fermentation broth .intended for isolation may comprise glycerol residues, propane-1,3-diol, impurities such as organic acids, including mainly: acetic acid, butyric acid, and carbohydrates (i.e mainly glucose, fructose, dextrose, xylose, arabinose, water-soluble starch fraction and cellulose residues): Presence of carbohydrates in the post-fermentation broth does not affect the efficiency of propane-1,3-diol extraction with 2-butanone.

The method ensures obtaining a desired concentration of propane-1,3-diol in the extract with high efficiency due to a possibility of recycling organic solvent used in the extraction process (either streamlined or stepwise). During extraction of propane-1,3-diol from post-fermentation broth no emulsion is formed at the interface between the phases, what usually happens in the case of solvents used previously (ethyl acetate, n-hexane, toluene, 4-methyl-2-pentanone) and considerably restricts, if not unables the use of such organic solvent in an industrial process.

The isolation is performed directly from the post-fermentation broth without any additional procedures, such as water evaporation (concentration) or inorganic salt addition (as in the method of CN101012151A). Only biomass removal from post-fermentation broth is obligatory and no other operations (like salting out) are needed. The isolation method of propane-1,3-diol from the post-fermentation broth requires low energy input. The obtained extract is solely further distilled under vacuum to acquire a final product with 99.90% purity. The method is short (two steps), inexpensive and based on unique properties of an organic solvent never applied before in such a simple method. Due to limitation of the number of isolation process steps and as a consequence, costs of propane-1,3-diol recovery from post-fermentation broth, the method of the invention is much more attractive than other yet known methods.

The object of the invention is presented in the following embodiments.

### EXAMPLE 1

Upon biomass removal by centrifugation the post-fermentation broth containing propane-1,3-diol (27.6g/L, 100mL) and 2-butanone (200(mL) were placed in the apparatus for continuous extraction (Soxhlet extractor for liquid-liquid extraction with organic solvent lighter than water). 2-butanone (150mL) was heated in a round-bottom flask for 1 h. Next the propane-1,3-diol content in organic and aqueous phases were determined. The continuous extraction of propane-1,3-diol was repeated 10 times for each fresh portion of 100 mL of post-fermentation mixture of propane-1,3-diol (27.6g/L) with the same amount of solvent (no replacement). Average yield of the extraction process was 91%. The resulting post-fermentation mixture was subsequently distilled under vacuum and the final product, propane-1,3-diol, was obtained with 99.90% purity. Depending on the given pressure (9-18 mmHg) the boiling point of propane-1,3-diol was varying in the range of 94-120°C.

### EXAMPLE 2

Upon biomass removal by centrifugation the post-fermentation broth containing propane-1,3-diol (35g/L, 100mL) and 2-butanone (200mL) were placed in the apparatus for liquid-liquid continuous extraction (Soxhlet extractor for liquid-liquid extraction with organic solvent lighter than water). 2-butanone (150mL) was heated in a round-bottom flask for 1 h. The propane-1,3-diol content in organic and aqueous phases were determined. The continuous extraction of propane-1,3-diol was repeated 10 times for each fresh portion of 100 mL of post-fermentation broth of propane-1,3-diol (35 g/L) with the same amount of solvent (no replacement). Average yield of the extraction process was 84%. The extract was subsequently distilled under vacuum and the final product, propane-1,3-diol, was obtained with 99.90% purity. Depending on the pressure (9-18 mmHg) the boiling point of propane-1,3-diol was varying in the range of 94-120°C.

### EXAMPLE 3

Upon biomass removal by centrifugation the post-fermentation broth containing propane-1,3-diol (60g/L, 100mL) and 2-butanone (200mL) were placed in the apparatus for liquid-liquid continuous extraction (Soxhiet extractor for liquid-liquid extraction with organic solvent lighter than water). 2-butanone (150mL) was heated in a round-bottom flask for 1 h. The propane-1,3-diol content in organic and aqueous phases were determined. The continuous extraction of propane-1,3-diol was repeated 10 times for each fresh portion of 100 mL of post-fermentation mixture of propane-1,3-diol (60 g/L) with the same amount of solvent (no replacement). Average yield of the extraction process was 73%. The extract. was subsequently distilled under vacuum and the final products propane-1,3-diol, was obtained with 99.90% purity.

Depending on the pressure (9-18 mmHg) the boiling point of propane-1,3-diol was varying in the range of 94-120°C.

### EXAMPLE 4

Upon biomass removal by centrifugation the post-fermentation broth containing propane-1,3-diol (35g/L, 100mL) and 2-butanone (200mL) were placed in the apparatus for liquid-liquid continuous extraction (Soxhlet extractor for liquid-liquid extraction with organic solvent lighter than water). 2-butanone (150mL) was heated in a round-bottom flask for 1,5 h. The propane-1,3-diol content in organic and aqueous phases were determined. The continuous extraction of propane-1,3-diol was repeated 10 times for each fresh portion of 100 mL of post-fermentation mixture of propane-1,3-diol (35 g/L) with the same amount of solvent (no replacement). Average yield of the extraction process was 90%. The extract was subsequently distilled under vacuum and the final product, propane-1,3-diol, was obtained with 99.90% purity.

Depending on the pressure (9-18 mmHg) the propane-1,3-diol boiling point was varying in the range of 94-120°C.

### EXAMPLE 5

Upon biomass removal by centrifugation the post-fermentation broth containing propane-1,3-diol (60g/L, 100mL) and 2-butanone (200mL) were placed in the apparatus for liquid-liquid continuous extraction (Soxhlet extractor for liquid-liquid extraction with organic solvent lighter than water). 2-butanone (150mL) was heated in a round-bottom flask for 2 h. The propane-1,3-diol content in organic and aqueous phases were determined. The continuous extraction of propane-1,3-diol was repeated 10 times for each fresh portion of 700 mL of post-fermentation mixture of propane-1,3-diol (60 g/L) with the same amount of solvent (no replacement). Average yield of the extraction process was 92%. The extract was subsequently distilled under vacuum and the final product, propane-1,3-diol, was obtained with 99.90% purity.

Depending on the pressure (9-18 mmHg) the propane-1,3-diol boiling point was varying in the range of 94-12°C.

### EXAMPLE 6

The results of lab-scale experiments were verified in pilot scale testing using extraction columns (Karr reciprocating column). Upon biomass removal by centrifugation the post-fermentation broth containing propane-1,3-diol (27g/L) and 2-butanone were placed in separated containers and pumped at the ratio of 1:7 (post-fermentation broth comprising propane-1,3-diol: organic solvent, v/v) into Karr reciprocating column in countercurrent flow. Column temperature range was 20-40°C. The process was carried out continuously. Average yield of extraction process was 80%.

### EXAMPLE 7

Upon biomass removal by centrifugation the post-fermentation broth containing propane-1,3-diol (27g/L) and 2-butanone were placed in separated containers and pumped at the ratio of 1:10 (post-fermentation broth containing propane-1,3-diol organic solvent, v/v) into Karr reciprocating column in countercurrent flow. Column temperature range was 20-40°C. The process was carried out continuously. Average yield of extraction process was 85%.

### EXAMPLE 8

Upon biomass removal by centrifugation the post-fermentation broth containing propane-1,3-diol (27g/L) and 2-butanone were placed in separated containers and pumped at the ratio of 1:15 (post-fermentation broth containing propane-1,3-diol : organic solvent, v/v) into Karr reciprocating column in countercurrent flow. Column temperature range was 20-40°C. The process was carried out continuously. Average yield of extraction process was 93%.

### EXAMPLE 9

Upon biomass removal by centrifugation the post-fermentation broth (containing propane-1,3-diol (27g/L) and 2-butanone were placed in separated containers and pumped at the ratio of 1:7 (post-fermentation broth containing propane-1,3-diol : organic solvent, v/v) into Scheibel extraction column in countercurrent flow. Column temperature range was 20-40°C. The process was carried out continuously. Average yield of extraction process was 77%.

### EXAMPLE 10

Upon biomass removal by centrifugation the post-fermentation broth containing propane-1,3-diol (27g/L) and 2-butanone were placed in separated containers and pumped at the ratio of 1:10 (post-fermentation broth containing propane-1,3-diol organic solvent, v/v) into Scheibel extraction column in countercurrent flow. Column temperature range was 20-40°C. The process was carried out continuously. Average yield of extraction process was 88%.

Based on the laboratory scale experiments it has been demonstrated that the time of propane-1,3-diol extraction process with organic solvent should be accordingly prolonged if the concentration of propane-1,3-diol in post-fermentation broth is increased to obtain similar process efficiency using the same amount of organic solvent.

With pilot scale experiments the process is controlled by the volume ratio of post-fermentation broth (containing propane-1,3-diol) to organic solvent.

## Claims

1. A method for isolation of propane-1,3-diol from the post-fermentation broth consisting in propane-1,3-diol extraction by means of a mixture of organic solvents **characterized in that** propane-1,3-diol is isolated, after previous biomass removal from post-fermentation broth, with 2-butanone that is present in the organic solvent mixture in an amount of minimum 10% or more.

2. The method according to Claim 1, **characterized in that said** method is carried out continuously and the organic solvent used in the extraction method is used repeatedly.

3. The method according to Claim 1, **characterized in that** said method is carried out stepwise and organic solvent used in the extraction process is used repeatedly.

4. The method according to any of Claims 1-3, **characterized in that** the volume ratio of post-fermentation broth, containing propane-1,3-diol, to the mixture of organic solvents is minimum 1:3.

5. The method according to any of Claims 1-4, **characterized in that** rafter previous biomass removal said isolation of propane-1,3-diol is conducted without salting out or concentration of post-fermentation broth.

6. The method according to any of Claims 1-5, **characterized in that** said isolation of propane-1,3-diol by means of organic solvent mixture, is followed by distillation under vacuum to obtain highly pure propane-1,3-diol.

7. The method according to any of Claims 1-6, **characterized in that** first the biomass is removed from post-fermentation broth comprising propane-1,3-diol by one of the methods: ultrafiltration, filtration, centrifugation or sedimentation, arid then the extraction of propane-1,3-diol with organic solvent mixture is carried out.

## Patentansprüche

1. Verfahren zur Isolierung von Propan-1,3-diol von der Fermentationsbrühe umfassend die Extraktion von Propan-1,3-diol mittels einer Mischung von organischen Lösemitteln, **gekennzeichnet dadurch dass** das Propan-1,3-diol nach der vorigen Entfernung der Biomasse aus der post-Fermentationsbrühe isoliert wird, mit 2-Butanon das in der Mischung organischer Lösemittel in einer Menge von mindestens 10% oder mehr enthalten ist.

2. Verfahren nach Anspruch 1, **gekennzeichnet dadurch, dass** das genannte Verfahren durchgehend ausgeführt wird, und das im Extraktionsverfahren eingesetzte organische Lösemittel wieder eingesetzt wird.

3. Verfahren nach Anspruch 1, **gekennzeichnet dadurch, dass** das genannte Verfahren schrittweise ausgeführt wird jedes im Extraktionsverfahren eingesetzte organische Lösemittel wieder eingesetzt wird.

4. Verfahren nach einem der Ansprüche 1 bis 3, **gekennzeichnet dadurch, dass** der Volumenanteil der post-Fermentationsbrühe, enthaltend Propan-1,3-diol zur Mischung der organischen Lösemittel mindestens 1:3 beträgt.

5. Verfahren nach einem der Ansprüche 1 bis 4, **gekennzeichnet dadurch, dass** nach dem vorherigen Entfernen der Biomasse die genannte Isolierung des Propan-1,3-diols mittels Aussalzen oder Konzentration der post-Fermentationsbrühe ausgeführt wird.

6. Verfahren nach einem der Ansprüche 1 bis 5, **gekennzeichnet dadurch, dass** der genannten Isolierung von Propan-1,3-diol mittels einer Mischung von organischen Lösemitteln, eine Destillation in Vakuumverhältnissen gefolgt wird, um hochreines Propan-1,3-diol zu erhalten.

7. Verfahren nach einem der Ansprüche 1 bis 6, **gekennzeichnet dadurch, dass** zuerst die Biomasse aus der das Propan-1,3-diol enthaltenden post-Fermentationsbrühe mittels eines der folgenden Verfahren entfernt wird: Ultrafiltration, Filtration, Zentrifugieren oder Sedimentation, und anschließend die Extraktion von Propan-1,3-diol mittels einer Mischung von organischen Lösemittel ausgeführt wird.

## Revendications

1. Un procédé pour l'isolement du propane-1,3-diol à partir du bouillon post-fermentation consistant à l'extraction du propane-1,3-diol au moyen d'un mélange de solvants organiques, **caractérisé en ce que** le propane-1,3-diol est isolé, après l'élimination préalable de la biomasse à partir du bouillon post-fermentation, avec la 2-butanone qui est présente dans le mélange de solvants organiques dans une quantité égale ou supérieure à 10% au minimum.

2. Le procédé selon la revendication 1, **caractérisé en ce que** ledit procédé est réalisé en continu et le solvant organique utilisé dans le procédé d'extraction est utilisé de façon répétée.

3. Le procédé selon la revendication 1, **caractérisé en ce que** ledit procédé est réalisé par étapes et le solvant organique utilisé dans le procédé d'extraction est utilisé de façon répétée.

4. Le procédé selon l'une quelconque des revendications 1 à 3, **caractérisé en ce que** le rapport volumétrique entre le bouillon post-fermentation, contenant du propane-1,3-diol, et le mélange de solvants organiques est 1 : 3 au minimum.

5. Le procédé selon l'une quelconque des revendications 1 à 4, **caractérisé en ce qu**'après l'élimination préalable de la biomasse, ledit isolement du propane-1,3-diol est réalisé sans relargage ou concentration du bouillon post-fermentation.

6. Le procédé selon l'une quelconque des revendications 1 à 5, **caractérisé en ce que** ledit isolement du propane-1,3-diol au moyen du mélange de solvants organiques, est suivi d'une distillation sous vide afin d'obtenir du propane-1,3-diol de haute pureté.

7. Le procédé selon l'une quelconque des revendications 1 à 6, **caractérisé en ce que** d'abord la biomasse est retirée du bouillon post-fermentation comprenant le propane-1,3-diol par une des méthodes: ultrafiltration, filtration, centrifugation ou sédimentation, et ensuite l'extraction du propane-1,3-diol avec le mélange de solvants organiques est réalisée.
